Europäisches Patentamt

European Patent Office

Office européen des brevets

(11) Veröffentlichungsnummer: **0 169 927**

**A1**

(12) **EUROPÄISCHE PATENTANMELDUNG**

(21) Anmeldenummer: **84109171.3**

(22) Anmeldetag: **02.08.84**

(51) Int. Cl.⁴: **A 23 L 3/34**
**A 23 L 3/00, A 61 L 2/20**

(43) Veröffentlichungstag der Anmeldung:
**05.02.86** Patentblatt **86/6**

(84) Benannte Vertragsstaaten:
**AT CH FR GB LI SE**

(71) Anmelder: **Neumayer, Ludwig**
**Friedrich-Ebert-Strasse 5C**
**D-8650 Kulmbach(DE)**

(72) Erfinder: **Neumayer, Ludwig**
**Friedrich-Ebert-Strasse 5C**
**D-8650 Kulmbach(DE)**

(74) Vertreter: **LOUIS, PÖHLAU, LOHRENTZ & SEGETH**
**Kesslerplatz 1 P.B. 3055**
**D-8500 Nürnberg(DE)**

(54) **Verfahren zur Entkeimung von mikrobiell kontaminierten Materialien.**

(57) Es wird ein Verfahren zur Entkeimung mikrobiell kontaminierter Materialien vorgeschlagen, bei dem das zu entkeimende Gut in Gegenwart eines Dampfes aus einem flüchtigen Alkohol, dem ggf. noch Wasserdampf zugesetzt wurde, erhitzt wird, wobei der Dampf mit einem inerten, mindestens annähernd auf die Dampftemperatur erhitzten Gas verdünnt und unter den angewandten Temperatur/Druck-Bedingungen neben der Dampfphase durch deren Kondensation eine Flüssigphase gebildet wird. Durch die Mitverwendung eines Verdünnungsgases ergeben sich wegen der Einsparung an Alkohol und Energie wirtschaftliche Vorteile. Durch die Begrenzung der Kondensatbildung kann eine Verbesserung der Qualität des zu entkeimenden Gutes erreicht werden, wenn in diesem durch Alkohol extrahierbare Bestandteile enthalten sind.

EP 0 169 927 A1

Croydon Printing Company Ltd.

"Verfahren zur Entkeimung von mikrobiell kontaminierten Materialien"

Die Erfindung bezieht sich auf ein Verfahren zur Entkeimung von mikrobiell kontaminierten Materialien, insbesondere pflanzlicher Herkunft, durch Erhitzen der Materialien
in Gegenwart von Alkoholdampf oder von Gemischen von Alkoholdämpfen, gegebenenfalls in Anwesenheit von Wasserdampf.

Das Erfordernis einer Entkeimung stellt sich häufig bei
pflanzlichen Materialien, aber auch bei anderen Produkten bzw. Gegenständen, vor allem dann, wenn die Produkte
zum Verzehr oder für die Herstellung von verderblichen
Lebens- oder Futtermitteln bestimmt sind. Als Beispiele
für solche, dem Keimverfall ausgesetzten pflanzlichen
Materialien seien Gewürze, pharmazeutische Drogen und Getreideprodukte (Zerealien) genannt. Eine Entkeimungsbehandlung ist z.B. auch für in der Pharmazie und Kosmetik verwendete Wirk- und Grundstoffe, z.B. chemische Agenzien,
Fette, Farbkörper, Füllstoffe, von Bedeutung. Schließlich
seien auch noch chirurgische Materialien (z.B. Catgut)

-1a-

und chirurgische Werkzeuge als Beispiele für die Anwendung von Entkeimungsprozessen genannt.

Für eine Entkeimungsbehandlung sind verschiedene Verfahren
bekannt. So werden Gewürze seit langem durch eine Behandlung mit Äthylenoxid entkeimt. Dabei kann die Keimzahl, die
bei dem naturbelassenen Erzeugnis 100 Millionen pro Gramm erreichen kann, bis unter 10.000 Keime pro Gramm gesenkt werden. Die allgemein praktizierte Anwendung von Äthylenoxid ist jedoch wegen der in dem Entkeimungsgut verbleibenden Rückstände an Äthylenoxid und der Entstehung von Reaktionsprodukten, wie Halogenhydrinen, also von Verbindungen mit mutagener Wirkung, nicht unproblematisch.

Eine Alternative zur Äthylenoxidbehandlung wäre die Behandlung des zu entkeimenden Gutes mit hochenergetischen Strahlen, z.B. Gamma- oder Elektronenstrahlen. Abgesehen davon, daß in manchen Ländern, so auch in der Bundesrepublik Deutschland, die Bestrahlung von Lebensmitteln bislang verboten ist, können sich als Folge der auftretenden Ionisierung unerwünschte stoffliche Veränderungen des behandelten Gutes einstellen.

Den gegen die vorgenannten Entkeimungsverfahren bestehenden Bedenken wird bei einer anderen bekannten Entkeimungsmethode dadurch Rechnung getragen, daß das betreffende Gut einer Hitzebehandlung in Gegenwart eines Dampfgemisches aus Wasser und einem flüchtigen Alkohol unterworfen wird. Bei der Entkeimung von Gewürzen wurden hierbei sehr gute bzw. völlig ausreichende Ergebnisse mit einem Äthanolanteil in dem Dampf von etwa 80 Gew.% erreicht. Aufgrund der Flüchtigkeit des verwendeten Alkohols lassen sich bei vertretbaren, d.h. verhältnismäßig niedrigen Temperaturen, genügend hohe Alkoholkonzentrationen in der Dampfphase erzielen. Falls dafür Sorge getragen wird, daß im Anschluß an die Hitzebehandlung der Alkohol durch Trocknung rückstandslos entfernt wird, kann auch die Verwendung von Methanol in Betracht gezogen werden. Weiterhin ist in diesem Zusammenhang Isopropanol als Beispiel für einen flüchtigen Alkohol zu nennen.

Bei der zuletzt genannten Methode hat sich weiterhin gezeigt, daß wesentlich geringere Temperaturen (ca. 100 bis 110°C) für die Erreichung des gewünschten mikrobiologischen

9. Verfahren nach einem der vorhergehenden Ansprüche, dadurch gekennzeichnet, daß das eingesetzte inerte Gas während der Entkeimungsbehandlung das Behandlungsgefäß durchströmt und außerhalb des Behandlungsgefäßes aufgeheizt wird.

10. Anwendung des Verfahrens nach einem der vorhergehenden Ansprüche zur Entkeimung von pflanzlichen Gewürzen.

11. Anwendung nach Anspruch 10, dadurch gekennzeichnet, daß die Entkeimung in Gegenwart eines aus pflanzlichen Gewürzen stammenden ätherischen Öls mit bakterizider Wirkung durchgeführt wird.

Effektes ausreichen als bei der Anwendung von trockener bzw. feuchter Hitze und auch die Erhitzungsdauer bei vergleichbaren Temperaturen erheblich verkürzt wird. Eine derartig schonende Behandlung ist für den Qualitätserhalt des behandelten Produktes wesentlich.

Das letztgenannte Verfahren besitzt wegen des verhältnismäßig hohen Alkoholverbrauchs zum einen wirtschaftliche, im Falle von pflanzlichen Materialien zum anderen aber auch verfahrenstechnische Nachteile, die sich negativ auf die Qualität des zu behandelnden Gutes auswirken. Da bei diesem Verfahren das zu entkeimende Gut durch dem Behandlungsgefäß (Autoklaven) zugeführten - kondensierenden - Dampf erhitzt wird, bildet sich eine verhältnismäßig große Menge an Kondensat auf dem zu entkeimenden Material. Es kondensiert hierbei nämlich solange Dampf auf dem Entkeimungsgut, bis es mindestens annähernd die Dampftemperatur erreicht hat, das System also im Gleichgewicht steht. Als Folge der starken Kondensatbildung kommt es während der Behandlung zu Extraktionsvorgängen, die besonders bei Gewürzen und pflanzlichen Drogen zu einer Beeinträchtigung der Qualität führen können und deshalb unerwünscht sind.

Der Erfindung liegt die Aufgabe zugrunde, das zuletzt beschriebene Verfahren unter Beibehaltung seiner Vorzüge so weiter zu entwickeln, daß es kostengünstiger durchgeführt und die Kondensatmenge im Vergleich zu dem bekannten Verfahren wesentlich verringert werden kann.

Die Lösung dieser Aufgabe besteht darin, daß bei dem eingangs genannten Entkeimungsverfahren, bei dem das zu entkeimende Gut in Gegenwart von Alkoholdampf oder von Gemischen von Alkoholdämpfen, gegebenenfalls in Anwesenheit

-4-

erhitzt wird
von Wasserdampf, der Dampf mit einem inerten, mindestens
annähernd auf die Dampftemperatur erhitzten Gas bzw. Gasgemisch verdünnt wird, wobei die Temperatur/Druck-Bedingungen so gestaltet werden, daß sich neben der Dampfphase durch deren Kondensation eine möglichst dünne Flüssigphase bildet.

Wenn im Zusammenhang mit der Erfindung von einem "inerten"
Gas (Gasgemisch) die Rede ist, dann sollen hierunter solche
Stoffe verstanden werden, die unter den Bedingungen der Entkeimungsbehandlung keine chemische Veränderung erfahren oder
im zu entkeimenden Gut bewirken. Als Beispiele für solche
Gase, die im Gegensatz zu den den Dampf (=Entkeimungsmedium)
bildenden Bestandteilen bei den Bedingungen der Entkeimungsbehandlung nicht kondensieren, seien Stickstoff oder Kohlendioxid oder Gemische hervon genannt.

Durch die Verdünnung des aus dem Dampf bestehenden Entkeimungsmediums mit dem inerten Gas können bei dem erfindungsgemäßen Verfahren Behandlungstemperatur und -druck von der
Zusammensetzung und Menge des eingesetzten Dampfes unabhängig
eingestellt werden, was bei der bisherigen Verwendung eines
nur aus Alkohol und Wasser gebildeten Sattdampfes wegen der
durch das Gleichgewicht zwischen Dampf- und Flüssigphase vorgegebenen Parameter nicht möglich war. Wenngleich bei Einsatz von Methanol der das Entkeimungsmedium bildende Dampf
vorzugsweise ausschließlich aus diesem Alkohol besteht wird
bei Verwendung anderer flüchtiger Alkohole, wie z.B. Äthanol
und/oder Isopropanol, dem Alkoholdampf vorzugsweise noch
Wasserdampf beigemischt, so daß dann auch die auf dem Entkeimungsgut gebildete Kondensatschicht aus einem Alkohol/
Wasser-Gemisch besteht.

Erfindungsgemäß können die für die Einstellung der Behandlungstemperatur erforderliche Energiezufuhr und der für
das Phasengleichgewicht benötigte Druck im wesentlichen
durch das inerte Verdünnungsgas erfolgen bzw. aufgebaut
werden. Dabei können die Temperatur/Druck-Bedingungen
unabhängig von der Menge des in das Behandlungsgefäß
eingebrachten Dampfes

so eingestellt werden, daß gerade nur so viel Kondensat
entsteht wie für die Bildung einer die Teilchen des zu
entkeimenden Gutes umschließenden (möglichst dünnen) Kondensatschicht erforderlich ist.

Bei Anwendung des erfindungsgemäßen Verfahrens wird durch
die Begrenzung der Kondensatmenge nicht nur der unerwünschte Extraktionseffekt weitgehend eliminiert, sondern auch
erheblich an Alkohol eingespart. Bezogen auf die Gesamtmenge des in dem Dampf enthaltenen Alkohol/Wasser-Gemisches
kann der Alkoholanteil 50-95 Gew.%, vorzugsweise 70-90 Gew.%,
betragen; besonders gute Entkeimungsergebnisse wurden mit
einem Alkoholanteil von 75-85 Gew.% erhalten. Bei Anwendung von Äthanol liegt das Optimum bei ca. 80 Gew.%. Bei
Verwendung von Methanol wurden sehr gute Ergebnisse erhalten, wenn dem Alkohol kein Wasser beigemischt war.

Was die Menge des eingesetzten Entkeimungsmediums (flüchtiger Alkohol ggf. mit Wasserzusatz) anbetrifft, so haben sich
1-10 Gew.%, bezogen auf die Menge des zu entkeimenden Gutes,
zur Erzielung befriedigender Entkeimungsergebnisse als ausreichend erwiesen. Demgegenüber wurde bei dem bekannten Verfahren mit einem Anteil von 15-20 Gew.% gearbeitet; bei ungenügender Beladung des Behandlungsgefäßes konnte für den
Aufbau des Behandlungsdrucks ein noch größerer Anteil erforderlich werden.

Wegen des Temperaturgefälles zwischen dem Dampf-und Gasraum
des Behandlungsgefäßes einerseits und dem zu behandelnden
Gut andererseits erweist es sich für die Einstellung von nur
kurzen Behandlungszeiten (2-5 Minuten) als zweckmäßig,das
Verdünnungsgas während der Behandlung laufend zu erhitzen
und umzuwälzen. Dabei kann die Aufheizung des Verdünnungsgases ausserhalb des Behandlungsgefäßes erfolgen. Die im
Einzelfall zur Anwendung kommende Behandlungstemperatur
richtet sich einmal nach dem Keimbefall. Zum anderen soll
sie auf eine möglichst schonende Behandlung des behandelten Gutes Rücksicht nehmen. In diesem Zusammenhang ist darauf hinzuweisen, daß es für die Entkeimungswirkung im allgemeinen günstiger ist, höhere Temperaturen während einer kürzeren Zeitdauer anzuwenden als niedrigere Temperaturen über eine längere Zeit. Die Anwendung des erfindungsgemäßen Verfahrens hat gezeigt, daß für die angestrebte Entkeimung im allgemeinen keine Behandlungstemperatur (= Temperatur in der Dampf- und Gasphase) von höher als 150°C erforderlich ist. Die bevorzugt zur Anwendung kommenden Temperaturen liegen zwischen 80 und 140°C. Für eine vorgegebene Behandlungstemperatur wird der Druck so eingestellt, daß sie dem Siedepunkt des Alkohols bzw. des Alkohol/Wasser-Gemisches entspricht oder darüber liegt.

Wenn sich trotz der Überschreitung der Temperatur des Siedepunkts des Entkeimungsmediums ein Gleichgewicht zwischen Dampf- und Flüssigphase einstellt, so findet dies seinen Grund darin, daß zwischen der Temperatur des zu entkeimenden Gutes und der Dampfphase ein Temperaturgefälle besteht. Die Behandlungsdauer ist zu kurz, als daß das in das Behandlungsgefäß eingebrachte, auf Raum- bzw. Umgebungstemperatur befindliche Entkeimungsgut auf dieselbe Temperatur wie der Dampf- und Gasraum gebracht werden könnte. Deshalb bildet sich auch dann noch Kondensat auf dem (kälteren) Entkeimungsgut wenn der Dampf des Entkeimungsmediums eine über dessen Siedepunkt liegende Temperatur aufweist.

Die Kondensation ist zu Beginn des Verfahrens, während dessen sich das Entkeimungsgut durch Wärmeabgabe des Entkeimungsmediums allmählich erwärmt, stärker als zum Ende hin. Dies hat zur Folge, daß der in dem Behandlungsgefäß herrschende Druck während des Behandlungsprozesses ansteigt. Die Verfahrensparameter (Druck/Temperatur) können dabei so eingestellt werden, daß die Menge der Kondensatbildung so begrenzt wird, daß sich auf den Teilchen des Entkeimungsguts nur eine diese umhüllende (dünne) Flüssigkeitsschicht ausbildet. Hierfür erweist es sich als zweckmäßig, wenn das Verdünnungsgas vor Einleitung in das Behandlungsgefäß bereits mindestens annähernd auf die Behandlungstemperatur (= Temperatur der Dampfphase) vorerhitzt wurde.

Dabei können die zur Anwendung kommenden Drücke mindestens zeitweise zwischen 1 (Atmosphärendruck) und 10 bar liegen, wobei vorzugsweise Drücke von zwischen 3 und 6 bar eingestellt werden. Erfindungsgemäß können dieselben, vergleichsweise flüchtigen Alkohole (z.B. Äthanol, Methanol, Isopropanol) eingesetzt werden wie bei dem bekannten Verfahren. Nach Beendigung der Behandlung kann das Entkeimungsmedium abgezogen und in einer Kühlfalle für die Wiederverwendung bei der Entkeimungsbehandlung einer nächsten bzw. anderen Charge aufgefangen werden. Bei der Entkeimungsbehandlung von Gewürzen wie beispielsweise Pfeffer, Ingwer, Kardamom, Chillies, Koriander, Kümmel, Senfsamen, Majoran, Muskat, können in dem rückgewonnenen Gemisch geringe Anteile an ätherischen Ölen enthalten sein. In den meisten Fällen hat sich bei der Wiederverwendung eines solchen wiedergewonnenen Gemischs für die Entkeimungsbehandlung eines anderen Gewürzes kein sensorischer Unterschied zu dem unbehandelten Gewürz gezeigt. Eine Reinigung des rückgewonnenen Alkohols vor seiner Wiederverwendung ist demnach nicht unbedingt notwendig. Verschiedentlich wurde bei der Wiederverwendung eines mit ätherischen Ölen angereicherten Alkohol/Wasser-Gemischs ein gegenüber dem reinen Gemisch besserer Entkeimungseffekt beobachtet. Es kann deshalb zweckmäßig sein, dem Entkeimungsmedium bereits zu Beginn ein ätherisches Öl mit bakterizider Wirkung beizugeben.

Bei Anwendung des erfindungsgemäßen Verfahrens, das auf die eingangs erwähnten Produkte und Gegenstände Anwendung finden kann, ergibt sich auch eine Energieeinsparung. Dies erklärt sich vor allem daraus, daß das Entkeimungsmedium zufolge des zugesetzten Verdünnungsgases eine vergleichsweise geringe Wärmekapazität aufweist.

Das vorgeschlagene Verfahren kann kontinuierlich oder als Batchprozess durchgeführt werden. Dabei wird das Entkeimungsgut zweckmäßig umgewälzt, um eine gute und gleichmäßige Kontaktierung mit dem Entkeimungsmedium zu erreichen. Hierfür kann das Behandlungsgefäß mit einer rotierenden Trommel ausgestattet sein.

Um ein Zusammenbacken des Entkeimungsgutes zu vermeiden und günstigere Voraussetzungen für die spätere Trocknung des Gutes zu schaffen, wie auch um eine Verflüchtigung von in dem Entkeimungsgut enthaltenem ätherischen Öl möglichst gering zu halten, wird das Gut zweckmäßig in unzerkleinerter, z.B. körniger, oder nur grob zerkleinerter Form eingesetzt. Bei Gewürzen kann eine der Entkeimungsbehandlung vorausgehende Zerkleinerung überhaupt im allgemeinen unterbleiben.

Ausführungsbeispiel 1

Für die Entkeimung von schwarzem Pfeffer mit einer Keimzahl von $3 \times 10^7$/g wurde ein Doppelmantelautoklav (ca. 50 l) verwendet. Durch die Wandbeheizung des Autoklaven sollte eine Kondensatbildung an der Gefäßwandung ausgeschlossen werden. Für die Umwälzung des später in den Autoklaven eingebrachten Entkeimungsgutes war das Druckgefäß mit einer Siebtrommel aus gelochtem Chrom-Nickel-Stahlblech ausgestattet, die sich mit einer Geschwindigkeit von 20 U/min. drehte. Der Autoklav stand mit einem Kessel für die Dampferzeugung in Verbindung.

Nach Vorheizung des Autoklaven und der Trommel auf etwa 103°C wurden 2 kg schwarzer Pfeffer (ganze Körner) eingebracht. Nach Verschließen des Druckgefäßes wurde bis 0,2 bar evakuiert, um die Luft, die explosive Gemische mit Äthanoldampf bilden könnte, zu entfernen und anschließend inertes Gas (Gemisch aus $CO_2$ und $N_2$) über einen Gaserhitzer solange dem Innenraum des Autoklaven zugeleitet, bis sich in diesem ein Druck von 3,1 bar aufgebaut hatte. Die Temperatur des zugeführten Gases lag bei 103°C.

Bei verschlossener Gaszuleitung wurde Dampf aus dem Dampfdruckkessel mit 80% Äthanol in der Gasphase (w/w) bis zu einem Druck von 3,5 bar injiziert. Der zufolge Kondensatbildung eingetretene Druckabfall wurde durch Zuführung geringer Mengen an dem vorerwähnten Verdünnungsgas ausgeglichen und somit über die gesamte Behandlungsdauer ein Druck von 3,5 bar aufrecht erhalten. Während der Hitzebehandlung des Entkeimungsgutes betrug die maximale Temperatur in der Gasphase 103°C, die im Gewürz 92°C.

Nach der sich über 12 Minuten erstreckenden Hitzebehandlung wurde das Dampf-Gasgemisch abgezogen und der Dampf zur späteren Wiederverwendung des Alkohol/Wasser-Gemisches in einer Kühlfalle aufgefangen. Dabei wurde bis auf 0,5 bar evakuiert, womit auch bereits eine Trocknung des Entkeimungsgutes eingeleitet wurde. Nach anschließendem Druckausgleich durch Belüften wurde das Gewürz entnommen und zur Endtrocknung noch einige Zeit offen an der Luft gelagert.

Aufgrund der in der Kühlfalle aufgefangenen Kondensatmenge errechnete sich ein Einsatz an Alkohol/Wasser-Gemisch von ca. 5% bezogen auf das Entkeimungsgut.

Die Keimzahl des in der beschriebenen Weise behandelten Pfeffers betrug nur noch $4 \times 10^3$/g.

0169927

-10-

Ausführungsbeispiel 2

------------------------

Statt des Äthanol/Wasserdampf-Gemisches im Beispiel 1 wurde Methanoldampf (ohne Wasserdampfzusatz) verwendet. Ansonsten wurde wie im Beispiel 1 verfahren mit folgenden Abweichungen

| | |
|---|---|
| Temperatur des vorgeheizten Autoklaven der Trommel | 99°C |
| Arbeitsdruck im Autoklaven | 2,8 bar |
| Temperatur des dem Autoklaven zugeführen inerten Gasgemischs | 100°C |
| maximale Temperatur der Gasphase während der Hitzebehandlung des Entkeimungsgutes | 98-99°C |
| maximale Temperatur des Gewürzes während der Hitzebehandlung | 91°C |
| Zeitdauer der Hitzebehandlung des Entkeimungsgutes | 10 Minuten |
| Druckabsenkung nach der Hitzebehandlung | bis auf 0,3-0,4 bar |
| Methanoleinsatz bezogen auf das Entkeimungsgut | ca. 7%. |

Das nach der Entkeimungsbehandlung anfallende Gewürz hatte eine Keimzahl von nur noch $2 \times 10^3$/g.

-1-

Patentansprüche
------------------

1. Verfahren zur Entkeimung von mikrobiell kontaminierten
   Materialien, insbesondere pflanzlicher Herkunft, durch
   Erhitzen der Materialien in Gegenwart von Alkoholdampf
   oder von Gemischen von Alkoholdämpfen, gegebenenfalls
   in Anwesenheit von Wasserdampf,
   d a d u r c h   g e k e n n z e i c h n e t ,
   daß der Dampf mit einem inerten, mindestens annähernd
   auf die Dampftemperatur erhitzten Gas bzw. Gasgemisch
   verdünnt wird, wobei die Temperatur/Druck-Bedingungen
   so gestaltet werden, daß sich neben der Dampfphase
   durch deren Kondensation eine möglichst dünne Flüssigphase bildet.

2. Verfahren nach Anspruch 1, dadurch gekennzeichnet,
   daß der Alkoholanteil 70-90 Gew.% bezogen auf die Gesamtmenge an Alkohol und Wasser, beträgt.

3. Verfahren nach Anspruch 1, dadurch gekennzeichnet, daß die Dampfphase aus Methanol besteht.

4. Verfahren nach einem der vorhergehenden Ansprüche, dadurch gekennzeichnet, daß die Gesamtmenge des Entkeimungsmediums aus Alkohol oder einem Alkohol/ Wasser-Gemisch 1-10 Gew.% bezogen auf die Menge der zu entkeimenden Materialien, beträgt.

5. Verfahren nach einem der vorhergehenden Ansprüche, dadurch gekennzeichnet, daß die Hitzebehandlung mindestens zeitweise bei einem Druck zwischen 1 und 10 bar, vorzugsweise 3 und 6 bar, und bei Temperaturen in der Dampf- bzw. Gasphase durchgeführt wird, welche dem Siedepunkt des Entkeimungsmediums bei dem jeweils angewandten Druck entsprechen oder darüber liegen.

6. Verfahren nach Anspruch 5, dadurch gekennzeichnet, daß die Temperaturen in der Dampf- bzw. Gasphase zwischen 80 und $150^{\circ}$ C, vorzugsweise um 100 und $140^{\circ}$ C liegen.

7. Verfahren nach einem der vorhergehenden Ansprüche, dadurch gekennzeichnet, daß das Entkeimungsgut auf eine Temperatur zwischen 80 und $125^{\circ}$C, vorzugsweise 90 und $120^{\circ}$ C, erhitzt wird.

8. Verfahren nach einem der vorhergehenden Ansprüche, dadurch gekennzeichnet, daß die zu entkeimenden Materialien während der Hitzebehandlung umgewälzt oder anderweitig bewegt werden.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-4 282 179  (D.A. GUNTHER)<br><br>* Zusammenfassung; Anspruch 1 *<br><br>--- | | A 23 L  3/34<br>A 23 L  3/00<br>A 61 L  2/20 |
| A | US-A-3 908 031  (H.E. WISTREICH et al.)<br>*   Zusammenfassung;   Ansprüche 1,5,9 *<br><br>--- | | |
| A | DE-A-2 921 706  (J. LAUFENBERG et al.)<br>* Anspruch 1 *<br><br>----- | | |

| RECHERCHIERTE SACHGEBIETE (Int. Cl.4) |
|---|
| A 23 L  3/00<br>A 61 L  2/00 |

Der vorliegende Recherchenbericht wurde für alle Patentansprüche erstellt.

| Recherchenort | Abschlußdatum der Recherche | Prüfer |
|---|---|---|
| BERLIN | 18-03-1985 | SCHULTZE D |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82